# EUROPEAN PATENT SPECIFICATION

(11) **EP 1 435 153 B1**
(45) Date of publication and mention of the grant of the patent: **03.05.2006**
(21) Application number: 02784076.8
(22) Date of filing: 10.10.2002
(51) Int. Cl.: H04L 12/28, H04L 12/46, H04L 12/56

(54) **METROPOLITAN AREA LOCAL ACCESS SERVICE SYSTEM**
DIENSTESYSTEM FÜR DEN LOKALEN ZUGRIFF IN EINEM METROPOLITANNETZ
SYSTEME DE SERVICES D'ACCES LOCAL DANS UN RESEAU METROPOLITAIN

(30) Priority: 11.10.2001 US 975474; 06.09.2002 US 228457
(43) Date of publication of application: 07.07.2004
(73) Proprietor: Onfiber Communications, Inc., Englewood, CO 80111 (US)
(72) Inventor: NIEZGODA, Paul, Austin, TX 78750 (US); STREET, Fraser, San Jose, CA 95128 (US)
(74) Representative: UEXKÜLL & STOLBERG
(86) International application number: PCT/US2002/032469
(87) International publication number: WO 2004/010653

(56) References cited:
- EXTREME NETWORKS: "Extreme Standby Router Protocol and Virtual Routing Redundancy Protocol" WHITE PAPER, [Online] 31 July 2002 (2002-07-31), pages 1-5, XP002243350 Retrieved from the Internet: <URL:http://builder.itpapers.com/whitepape rs/> [retrieved on 2003-06-03]
- ANRITSU: "VRRP Application Note" INTERNET, [Online] May 2000 (2000-05), pages 1-4, XP002243351 Retrieved from the Internet: <URL:http://www.us.anritsu.com/downloads> [retrieved on 2003-06-04]
- KNIGHT S ET AL: "Virtual Router Redundancy Protocol" INTERNET, April 1998 (1998-04), XP002135272 Retrieved from the Internet: <URL:ftp://ftp.isi.edu/in-notes/rfc2338.tx t> [retrieved on 2000-04-10]
- LI T ET AL: "RCF 2281 - Cisco Hot Standby Router Protocol (HSRP)" INTERNET, March 1998 (1998-03), XP002196799 Retrieved from the Internet: <URL:http://kaizi.viagenie.qc.ca/ietf/rfc/ rfc2281.txt> [retrieved on 2002-04-19]
- "ExtremeWare" EXTREME NETWORKS PRODUCT INFORMATION SHEET, [Online] 1999, XP002243863 Retrieved from the Internet: <URL:http://www.extremenetworks.com> [retrieved on 2003-06-11]

## Description

### FIELD OF THE INVENTION

The invention generally relates to the field of fiber optic communications networks, and more particularly to a robust network having a network failover transition system to permit continued data communication in the event of primary network failure.

### RELATED ART

The growth of the Internet has created unprecedented demand for high-speed broadband connectivity in telecommunications networks. However, access connections between corporate Local Area Networks ("LANs") and existing service provider networks, such as those operated by long-haul carriers and Internet Service Providers ("ISPs"), generally have been limited to relatively slow, hard to provision T1 (1.5 Mbps) or DS-3 (45 Mbps) data speeds due to infrastructure limitations in most metropolitan areas.

The lack of bandwidth throughout metropolitan areas is a function, principally, of two independent factors. First, there is a deficiency in high speed fiber optic access rings and/or fiber optic "tails" into major buildings in metro areas. Second, the existing metropolitan area carriers continue to use older, installed SONET (Synchronous Optical NETwork) architecture which, although it allows data streams of different formats to be combined onto a single high speed fiber optic synchronous data stream, cannot be scaled to meet future bandwidth requirements. Although customer demand for increased bandwidth has been growing at exponential rates, there is a mismatch between carrier long-haul backbones and metro area backbones on the one hand and local loop access on the other hand. Despite the aggressive deployment of fiber-optic networks nationwide, relatively little fiber has been deployed in the local access market or "last mile." Fiber deployment in Metro Area Networks ("MANs") has been primarily to carrier and service provider locations, or to a relatively small number of very been primarily to carrier and service provider locations, or to a relatively small number of very large commercial office building sites. At the current time, it is estimated that as few as 10% of all commercial buildings in the United States are served with fiber-optic networks.

Currently, most local connectivity service provides are primarily providing SONET-based services and are investing little in the services required for expanded local connectivity-e.g., Ethernet and Wavelength services. In general, existing local service providers have not moved forward to upgrade local fiber infrastructure to support these latter services.

In order to provide compatibility and easy upgrading from existing services to new services, it is desirable to provide SONET, Ethernet and Wavelength services in the metropolitan and access segments of the communications infrastructure, making use of a common interface system and fiber optic cables. In this way, it is possible for customers to migrate smoothly and at an opportune time from the traditional SONET-based circuits to Ethernet circuits and, possibly, to transparent wavelengths. Such an evolutionary connectivity path enables customers to access the right amount of bandwidth at the right time.

In ANRITSU: 'VRRP Application Note" INTERNET [Online] May 2000 (2000-05), pages 1-4, XP002243351, relates to network failover transition system of the type as defined in the preamble of claim 1. Disclosed therein is a redundant router protocol to increase reliability of existing networks. Whereas this sort of redundancy is useful, it does not teach the master and standby modes and auto-negotiation of the present invention.

### SUMMARY OF THE INVENTION

A network failover transition system and method according to the invention are set out in claim 1 and in claim 10.

In connection with the present invention, a service network provides customers with a highly-available transparent Layer 2 network connection between their edge IP equipment and their subscribers' edge IP equipment.

Layer 2, known as the bridging or switching layer, allows edge IP equipment addressing and attachment. It forwards packets based on the unique Media Access Control ("MAC") address of each end station. Data packets consist of both infrastructure content, such as MAC addresses and other information, and end-user content. At Layer 2, generally no modification is required to packet infrastructure content when going between like Layer 1 interfaces, like Ethernet to Fast Ethernet. However, minor changes to infrastructure content not end-user data content may occur when bridging between unlike types such as FDDI and Ethernet. Additionally, the Ethernet service can inter-connect customers to create an "extended" LAN service.

Layer 3, known as the routing layer, provides logical partitioning of subnetworks, scalability, security, and Quality of Service ("QoS"). Therefore, it is desirable that the network remain transparent to Layer 3 protocols such as IP. This is accomplished by the combination of a particular network topology combined with failure detection/recovery mechanisms, as more fully described herein.

Further features and advantages of the invention will appear more clearly from a reading of the detailed description of the preferred embodiments of the invention, which are given below by way of example only, and with reference to the accompanying drawings, in which like references indicate similar elements, and in which:

### BRIEF DESCRIPTION OF THE DRAWINGS

**Fig. 1** is a schematic diagram of a local distribution portion of an overall fiber optic network, illustrating the relationship between multiple subscribers disposed on collection loops connected to a hub facility via a feeder loop;
**Fig. 2** is a schematic diagram illustrating a typical longest path around an access distribution network;
**Fig. 3** is a schematic diagram illustrating an alternative design with nested feeders;
**Fig. 4** is a schematic diagram of a dual overlay ring topology within the core;
**Fig. 5** is a schematic diagram of a working path and a protection path across the core connecting a subscriber's Layer 3 switch to its carrier/ISP; and
**Fig. 6** is a simplified logical diagram of the end-to-end Ethernet service indicating where ESRP is utilized.

### DETAILED DESCRIPTION OF THE PREFERRED EMBODIMENT

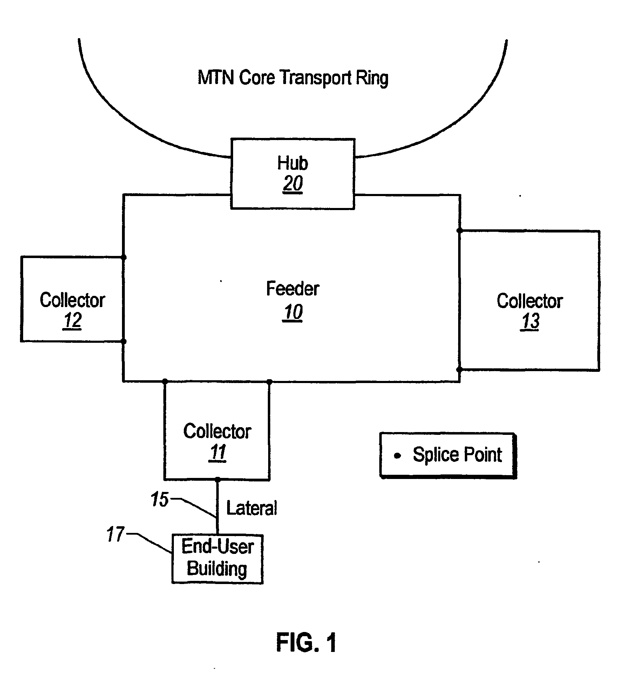

A fiber optic transport network can generally be described in terms of three primary components: (i) a leased transport network (LTN), (ii) a leased distribution network (LDN); and (iii) a built distribution network (BDN), which may be a distribution network in accordance to the present invention (*see* **Figs. 1-6**).

The LTN is the main transport layer of each metropolitan system. It typically consists of a high-bandwidth, flexible DWDM transport pipe used to connect customer locations (such as data centers, co-location hotels, and large customer POPs) to distribution networks.

The distribution networks may comprise both LDN and BDN designs, though either may be excluded. Although similar in general purpose, an LDN and a BDN may use differing architectural approaches to bring traffic to the LTN. While the LDN typically relies on TDM (and sometimes WDM) electronics to multiplex traffic onto limited quantities of fiber, the distribution network according to the present invention uses larger quantities of fiber, enabling a reduced reliance upon multiplexing electronics.

The following description will focus specifically on the architectural design and operation of a distribution network especially suitable for a BDN, though it may have other applications; particularly to an LDN. Detailed discussions of LTN and LDN designs may be found in other publicly available documents. The distribution network architecture maximizes the saturation of the potential subscriber base at minimal expense and is designed with the following criteria in mind.

Each subscriber should have access to a route-diverse connection to the LTN hub. In a preferred embodiment, these connections are capable of supporting:
(1) SONET services that require Line Overhead termination and Automatic Protection Switching (APS) controlled by the distribution network (DS-3, OC-3/OC-3c, OC-12/OC-12c, and OC-48/OC-48c).
(2) Data devices with SONET interfaces that require Line Overhead termination, but may lack APS functionality (DS-3, OC-3c, OC-12c, and OC-48c).
(3) Ethernet services (10, 100, and 1000 base).
(4) Wavelength services (1000-LX/LH/ZX, OC-48/OC-48c).

The distribution design is scalable and flexible enough to adapt to the eventual traffic needs of the network. Circuits from multiple subscribers should be reasonably segregated. Where feasible, the distribution architecture should ensure that work requested by one subscriber seldom impacts other subscnbers.

Referring now to **Fig. 1**, the distribution network comprises a major feeder ring **10** with a series of smaller, subtending collector rings **11 - 13**. In a common metropolitan-wide network design, collector rings are installed to follow city streets. Feeder ring **10** accesses at least one LTN Hub **20,** where the distribution network fiber may be terminated to high-density fiber distribution panels (FDPs).

One particular feature of any local distribution architecture is the quantity of fiber run on the distribution network. Although fiber counts will vary based on the logistics of the distribution area, a typical feeder ring **10** will contain 432 fibers, and typical collectors **11-13** each will contain 144 fibers. Laterals (e.g., 15) extend from the collector rings **11-13** to subscriber buildings (e.g., **17**), and will typically contain 48 fibers. As shown, each collector (**11-13**) is preferably deployed with two splice points to the feeder **10.** A person of ordinary skill in the art will readily appreciate that fiber counts may be varied upwardly or downwardly without deviation from the present invention. The overall goal of the preferred embodiment is to provide, for each subscriber, optical service with at least one diversely-routed, dedicated fiber pair.

The described distribution network architecture has the following advantages over conventional TDM and WDM distribution networks.
(i) Lower cost - At the present time, the major cost associated with any new fiber run is the cost of opening and closing the trench. Since this cost is substantially ' independent of the number of fibers being run, the comparison between a bulk-fiber distribution network and a TDM-based distribution network (similar to the LDN design), for example, becomes mainly a comparison between costs of fiber versus electronics. On relatively short fiber runs (like a distribution network), additional fiber is generally less expensive than TDM electronics. When the costs associated with space, power, operation, maintenance, and management of the TDM electronics is added, the cost advantages of a bulk-fiber approach increase dramatically.
(ii) Manageability - Connecting subscribers to a distribution network becomes a relatively simple task of splicing fibers between the subscriber building and the collector. This design eliminates an extra layer of TDM circuit provisioning and management. Requirements of TDM software upgrades and equipment failures are likewise reduced.

### Fiber Plant

At present, the majority of optical circuits transported over the distribution network preferably will utilize 1310nm lasers and therefore, Non-Dispersion Shifted Fiber (NDSF) is the preferred fiber for such distribution network deployment. Non-Zero Dispersion Shifted Fibers (NZ-DSF) and Multi-Mode Fiber (MMF), though not presently preferred, may be used in alternative embodiments.

### Subscriber Laterals

Normally, a 48-count fiber bundle can be run in a single 38.1 mm (1.5") conduit between the collectors **11-13** and subscriber facilities. As a result, most laterals will be single-threaded. A person of ordinary skill in the art will readily appreciate that dual-threaded laterals, and laterals of different fiber counts, may also be run. Depending on system requirements, fusion or mechanical splices may be utilized. Mechanical splices are preferably used between the lateral and the Collector fibers. High quality mechanical splices can be obtained that provide typical insertion loss below 0.10dB. Fusion splices are preferably utilized between the lateral and the FDP within the subscriber site. Fusion splices can routinely introduce insertion losses of less than 0.05dB.

### Collector Loops

In a preferred embodiment, a collector loop will consist of a 144-count fiber bundle run in a single 102 mm (4") conduit. The 4" collector can compartmentalized, such as with individual 25.4 mm (1") conduits or "MaxCell"® fabric inner ducts. In cases where a single Collector runs in the same trench as a Feeder loop, it is expected that the Collector fibers will utilize one of the Feeder's expansion conduits instead of the 102 mm (4") conduit discussed above. Both ends of a Collector loop will not necessarily intersect the Feeder at the same physical location. Fusion splices are preferably utilized between the Collector and Feeder loops.
(iii) Scalability - Since each subscriber's optical service may be on a dedicated fiber pair, significant capacity exists at the outset and there is no concern regarding TDM circuit fill ratios or provisioning anomalies. This design also minimizes the need for TDM reconfigurations to support capacity expansions.
(iv) Circuit protection - Isolating each subscnber's optical service on a dedicated fiber pair reduces the possibility that work requested by one subscriber affects other subscribers. This represents a significant advantage in network accessibility when compared to designs that rely on multiple subscribers sharing a TDM resource.

Although a primary goal of the preferred embodiment of the BDN design is to reduce the use of electronics at each subscriber site, electronic components will still be required for subscribers who elect to use electrical circuits (e.g., DS-3, 10-base, and 100-base). Electrical circuits must still be converted into optical circuits for transport around the BDN. Due to the distances within the BDN, single-mode fiber connectivity is the preferred embodiment to support the connection between the subscriber site and the hub location. Therefore, additional electronics may be required for subscribers who desire optical circuits when these subscribers occupy locations or operate equipment with an embedded base of Multi-Mode Fiber ("MMF").

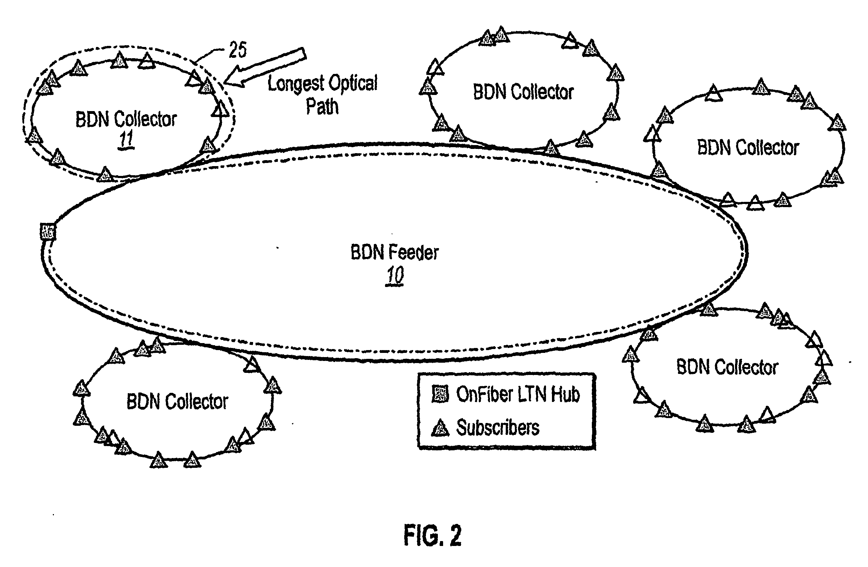

**Fig. 2** illustrates the longest optical path **25** around the distribution network. This calculation is the sum of the length of the longest collector (shown as **11**) and the length of the feeder **10**. The longest optical path **25** is a significant limitation to be considered in the design of the distribution network, as discussed in greater detail below.

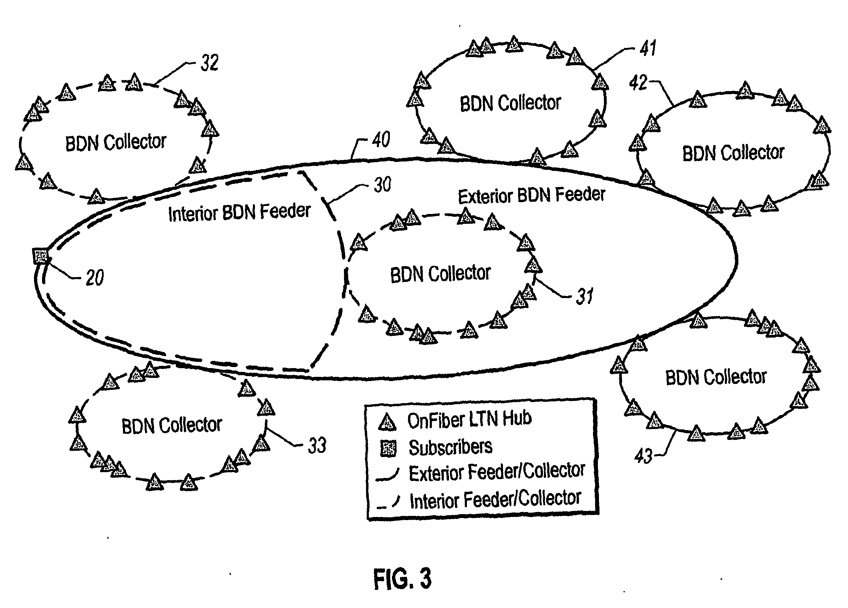

The physical connections of circuits and facilities on the distribution network are described in greater detail below. Exemplary subscriber connections can be found by reference to **Figs. 3 - 7,** discussed below.

### LTN Hubs

At LTN Hub **20** locations, distribution network fiber can be terminated to high-density Fiber Distribution Panels (FDPs). From these locations, subscriber circuits may be cross-connected to ADM equipment, Ethernet switches, or directly to an LTN DWDM system. The ADMs and Ethernet switches aggregate circuits with common destinations (e.g., customer locations) and transfer them to the LTN for transport around the metropolitan network.

### Single-Tenant Subscriber Facilities

In a single-tenant subscriber facility, a lateral fiber offshoot can be deployed to connect the appropriate feeder **10** fibers to a low-density FDP on the subscriber's premises. For optical services, this FDP will serve as a demarcation point between the distribution network and the subscriber equipment. For electrical services, an additional component can be placed at the subscriber's site. This component typically will be a media converter capable of converting an electrical signal into a higher-rate optical signal for transport over the distribution network. This converter equipment can usually be powered by the subscriber's AC power facilities, although a small UPS (Uninterruptible Power Supply) device may be required in cases where brownout protection is lacking from the subscnber's AC feed.

### Multiple-Tenant Subscriber Facilities

Access to multiple-tenant facilities may be similarly designed. A primary difference will often be the equipment location. Any necessary auxiliary electrical equipment (FDP, DSX, patch panel, SONET TDM, Ethernet switch, media converter) may be located either within a Minimum Point of Entry (MPOE) facility inside the building or within the subscriber's location. When it is located within the MPOE, such equipment preferably should be within a protected enclosure (e.g., a cage or locked cabinet). DC power (e.g., -48V regulated with battery reserve) may be provided as an option in larger MPOE facilities. However, AC power with a UPS reserve is also feasible.

In order to minimize the frequency of adding new splices between Collector and Feeder loops, a reasonable quantity of splices will be generated at the outset to cover the near-term growth of traffic on the distribution network.

### Feeder Loops

In most cases, feeder loop 10 will consist of a 288 or 432-count fiber bundle run in a single 38,1 mm (1.5") conduit. A person of ordinary skill in the art will readily appreciate that fiber bundles of greater or lesser count may be used as appropriate. Additional conduits preferably will be included along the Feeder path to accommodate future growth. In cases where a Collector loop runs parallel to a Feeder loop, it is expected that the Collector will utilize one of the Feeder's surplus 38,1 mm (1.5") conduits instead of the Collector's usual 102 mm (4") conduit. Fusion splices should be utilized for all connections to and from Feeder loops. All fusion splices should introduce an insertion loss of no greater than 0.05dB.

Feeder **10** fibers can be spliced to pigtails and terminated in the Hub **20** location on initial installation. This reduces the frequency of adding new splices on the feeder loop **10** and reduces the interval required for service activation.

### An Alternative Embodiment-Additional Equipment

In this embodiment, in addition to the conventional feeder/collector architecture, additional electronic equipment can be deployed at either the subscriber facility or the hub 20 to provide intermediate-reach optics on both sides of the transmission link.

For example, with respect to SONET equipment, a series of ADMs will already exist at the hub locations to aggregate subscriber traffic, and IR-1 optics can be supported on each optical interface of the ADMs. Wavelength services pose a more complex problem. Since these services enter the DWDM directly at the Hub, they are limited by the current SR client-side interface on the DWDM equipment. Since it is unlikely that any wavelength service below an OC-48 or Gigabit Ethernet data rate will be used in this context (as this would require dedicating a DWDM wavelength to an OC-3 or OC-12 rate circuit), this would only pose a problem for OC-48 or Gigabit Ethernet wavelength services.

In the case of Gigabit Ethernet services, upgrading a subscriber to a GBIC equivalent to the Finisar 1319-5A-30 would improve the optical reach to roughly 26.2 km (16.3 miles). This is less than one mile shorter than the range of a bi-directional IR-1 link. The OC-48/OC-48c case is more difficult. To support this service, a subscriber positioned near a Hub either should use LR-1 optics (assuming they are available on the subscriber equipment), or place an OC-48 regenerator at the Hub location.

### Second Alternative Embodiment-Nested Feeders

In this scenario, the distribution network provider deploys a pair of nested Feeder rings **30** in each distribution network. The collectors **31, 32** and **33** closest to the hub **20** are placed on the nested feeder **30,** while the collectors **40, 41** and **42** located farther out are placed on the longer feeder **40. Fig. 3** displays a generic example of this configuration. With the **Fig. 3** type of configuration, the longer feeder **40** can remain longer (e.g., more than 11.3 km (7 miles) in circumference) without stranding capacity because the collectors closest to the LTN hub 20 have a shorter path available to them.

Although the additional cross-section of fiber that completes the interior Feeder may increase the cost of the distribution network, it may also provide the opportunity to place one or more additional Collectors that would have otherwise been difficult to attach to the single Feeder design.

### Decision Rule for Distribution Network Variants

In significant part, the distribution network design can be directed based on the guidelines below. In each case, the longest subscriber path is calculated as follows. Each Collector has a corresponding longest circuit path. The longest circuit path can be defined as the sum of the circumference of the Collector and the longest route around the Feeder between the Collector and the Hub. This value represents the maximum distance that a subscriber circuit on that Collector can possibly travel en route to the Hub. This value is unique to each Collector on the distribution network. After calculating this value for each collector on the distribution network, the largest of these values would represent the longest subscriber path on that distribution network.

### Longest Subscriber Path is less than 14.5 km (9 Miles)

Any distribution network that meets this requirement can be designed using the conventional single Feeder, multiple Collector architecture.

### Longest Subscriber Path is between 14.5 km and 25.7 km (9 and 16 Miles)

Any distribution network that falls in this category will encounter complications based on the optical link budget. With this in mind, the distribution network should be examined in detail to determine whether a nested feeder approach is appropriate. In most cases, the nested feeder architecture is desirable when a significant portion of potential subscribers must traverse more than nine miles of fiber (longest route around the Feeder) to access the Hub or the additional cross section of fiber added to create an Interior Feeder allows the addition of a new, desirable Collector that would have otherwise been inaccessible.

### Longest Subscriber Path is greater than 25.7 km (16 Miles)

Any distribution network in this classification gives rise to design problems as one begins to exceed the limits of both Gigabit Ethernet and SONET IR-1 optics. In this case, either the distribution network may be configured to utilize the nested feeder architecture, or it can be redesigned to shorten the longest subscriber path.

### Synchronization

Subscribers purchasing SONET services can synchronize their equipment with the Network by line-timing from the optics of the ADM at the Hub. Similarly, subscribers purchasing Ethernet services can line-time from the optics of the system Ethernet Switch at the Hub facility. However, this option is not available for wavelength services, as these circuits bypass any equipment that can connect to a BITS clock. Subscribers who desire wavelength services must therefore either provide their own clock source or line-time from the customer equipment that they logically attach to on the far end of the distribution network. Should either of these options be unavailable for a given subscriber circuit, there is still a likely option available to provide error-free service. Depending on the age of the equipment, Telcordia compliant devices should contain an internal SONET Minimum Clock (SMC) source or Stratum 3 clock source. Either should provide adequate synchronization for SONET signals. Any equipment free-running on a Stratum 3 or SMC source should operate error-free under normal conditions. The major perceptible difference will be an increase in the frequency of pointer justification events between interconnected devices.

Depending on the situation, SONET equipment installed at a subscriber site may be owned and maintained either by the distribution network operator or by the individual subscriber. Ethernet equipment installed at a subscriber site will generally be owned and maintained by the subscriber.

All distribution network electronics installed at subscriber locations that are owned and maintained by the distribution network operator should be remotely manageable, and should be capable of forwarding alarm messages to the system NOC. SONET equipment will commonly utilize the SONET Section Data Communications Channel (SDCC) to communicate with the ADM equipment installed at the Hub.

### The Ethernet Services Network

### Resiliency

It is desirable that a network of the type described herein be substantially always available. In addition, a desirable network architecture will provide fast recovery from failure to meet uptime objectives. Taking as an example Ethernet as the local loop technology, it is an objective that Ethernet services be highly available. This objective makes the elimination of any Spanning Tree Protocol ("STP") from the architecture desirable. In a preferred embodiment, STP is not used because otherwise, network recovery times may be of the order of minutes per failure.

The network elements which provide redundancy need not be co-located with the primary network elements. This design technique reduces the probability that problems with the physical environment will interrupt service. Problems with software bugs or upgrades or configuration errors or changes can often be dealt with separately in the primary and secondary forwarding paths without completely interrupting service. Therefore, network-level redundancy can also reduce the impact of non-hardware failure mechanisms. With the redundancy provided by the network, each network device no longer needs to be configured for the ultimate in standalone fault tolerance. Redundant networks can be configured to fail-over automatically from primary to secondary facilities without operator intervention. The duration of service interruption is equal to the time it takes for fail-over to occur. Fail-over times as low as a few seconds are possible in this manner.

### Dual Physical Overlay Ring Core Topology

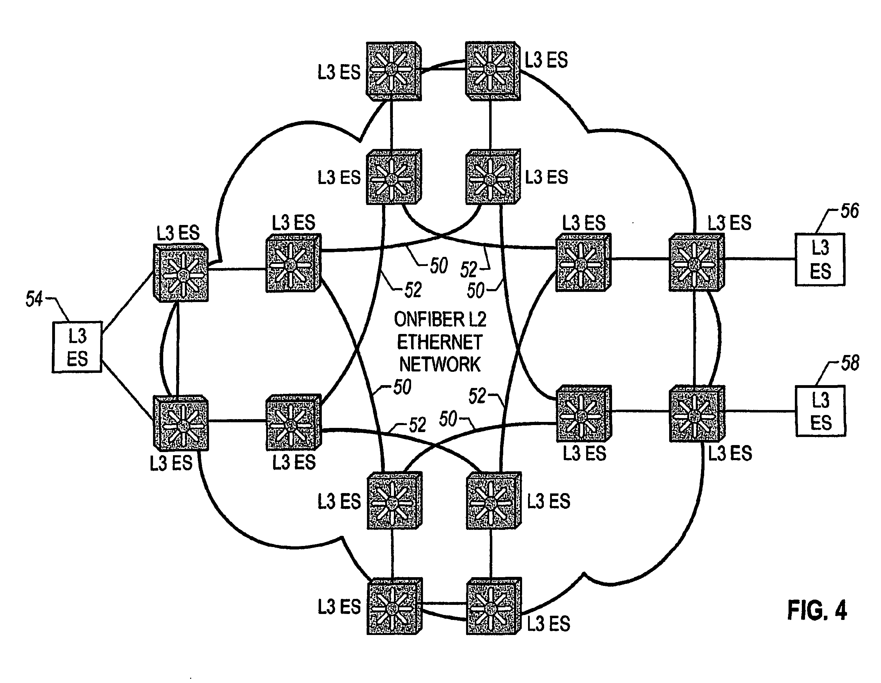

The local services network (e.g., Ethernet) according to the preferred embodiment of the present invention comprises a dual overlay ring topology within the core. This topology is shown in **Fig. 4**. As can be seen, the dual overlay ring topology is a physical topology in which two complete physical paths are disposed to ensure that two data channels are available during normal periods of use so that at least one is available to communicate information in the event the other becomes unavailable.

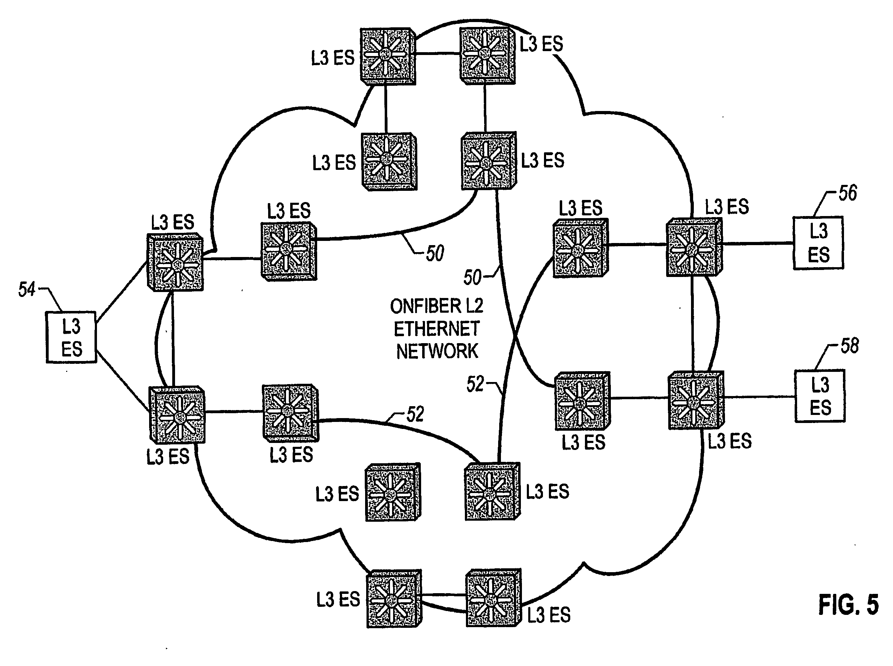

This physical topology allows the creation of a working path **50** and a protection path **52** across the network connecting each subscriber (L3 Switch **54**) to their carrier/ISP (L3 Switches **56, 58**). The working path **50** can be provisioned on one ring while the protection path **52** can be provisioned on the other ring shown, creating the logical connectivity topology shown in **Fig. 5.**

Logical connectivity may be accomplished in many ways, such as by using Ethernet Virtual LAN (VLAN) tagging, as defined in the IEEE 802.1Q standard. A VLAN can be roughly equated to a broadcast domain. More specifically, VLANs can be seen as analogous to a group of end-stations, perhaps on multiple physical LAN segments, which are not constrained by their physical location and can communicate as if they were on a common LAN. The 802.1Q header adds two octets to the standard Ethernet frame. By configuring ports on the Ethernet switches (e.g., **54**) to be part of the specific customer's VLAN, the logical connectivity paths are created through the network. This process is somewhat analogous to creating a Permanent Virtual Circuit ("PVC") in the Frame Relay or ATM environment

Extreme Network's Standby Router Protocol ("ESRP") may be used to detect and recover from failures that occur within the Ethernet Network. Additional protocols may be implemented to support detection and recovery of failures that occur at the Carrier/ISP connection. Some of these protocols are Hot Standby Router Protocol ("HSRP") and Virtual Router Redundancy Protocol ("VRRP"). Note that standard Layer 2 protection protocols such as 802.1D Spanning Tree are not required in some embodiments of the present invention.

### Overview of ESRP

ESRP is a feature of the Extreme OS (operating system) that allows multiple switches to provide redundant services to users. In addition to providing Layer 3 routing redundancy for IP, ESRP also provides Layer 2 redundancy. The Layer 2 redundancy features of ESRP offer fast failure recovery and provide for a dual-homed system design generally independent of end-user attached equipment.

ESRP is configured on a per-VLAN basis on each switch. This system utilizes ESRP in a two switch configuration, one master and one standby. The switches exchange keep-alive packets for each VLAN independently. Only one switch can actively provide Layer 2 switching for each VLAN. The switch performing the forwarding for a particular VLAN is considered the "master" for that VLAN. The other participating switch for the VLAN is in 'standby' mode.

For a VLAN with ESRP enabled, each participating switch uses the same MAC address and must be configured with the same IP address. It is possible for one switch to be master for one or more VLANs while being in standby for others, thus allowing the load to be split across participating switches.

To have two or more switches participate in ESRP, the following must be true. For each VLAN to be made redundant, the switches must have the ability to exchange packets on the same Layer 2 broadcast domain for that VLAN. Multiple paths of exchange can be used, and typically exist in most network system designs that take advantage of ESRP. In order for a VLAN to be recognized as participating in ESRP, the assigned IP address for the separate switches must be *identical.* ESRP must be enabled on the desired VLANs for each switch. Extreme Discovery Protocol (EDP) must be enabled on the ports that are members of the ESRP VLANs.

### Master Switch Behavior

If a switch is master, it actively provides Layer 2 switching between all the ports of that VLAN. Additionally, the switch exchanges ESRP packets with other switches that are in standby mode.

### Standby Switch Behavior

If a switch is in standby mode, it exchanges ESRP packets with other switches on that same VLAN. When a switch is in standby, it does not perform Layer 2 switching services for the VLAN. From a Layer 2 switching perspective, no forwarding occurs between the member ports of the VLAN. This prevents loops and maintains redundancy.

### ESRP Tracking

ESRP can be configured to track connectivity to one or more specified VLANs as criteria for fail-over. The switch that has the greatest number of active ports for a particular VLAN takes highest precedence and will become master. If at any time the number of active ports for a particular VLAN on the master switch becomes less than the standby switch, the master switch automatically relinquishes master status and remains in standby mode.

Additionally, ESRP can be configured to track connectivity using a simple ping to any outside responder (ping tracking). The responder may represent the default route of the switch, or any device meaningful to network connectivity of the master ESRP switch. It should be noted that the responder must reside on a different VLAN than ESRP. The switch automatically relinquishes master status and remains in standby mode if a ping keep-alive fails three consecutive times.

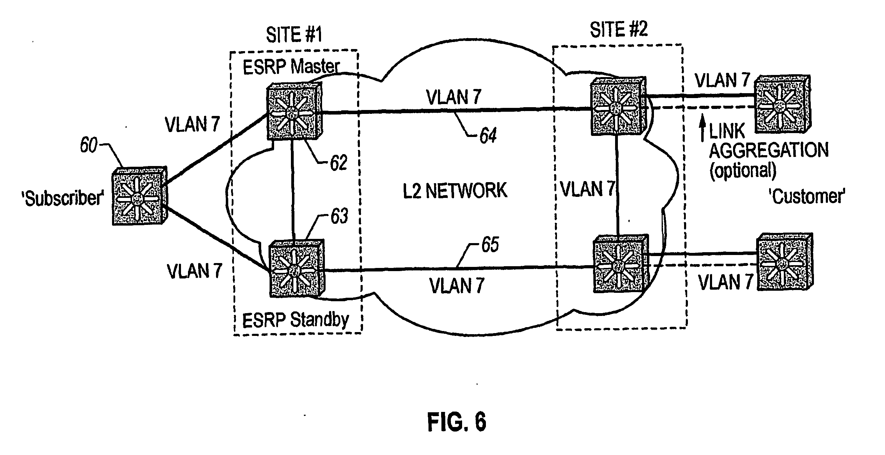

A simplified drawing of the logical topology is shown in **Fig. 6**, indicating where ESRP is utilized in the present distribution network design. **Fig. 6** depicts ESRP enabled in the switches (**62, 63**) directly attached to the subscriber **60**. Port track is used to detect local failure of a link directly connected to these switches while ping track is used to detect core network failures. If a failure is detected anywhere along the active path 64, ESRP will failover to allow traffic to flow on the standby path **65**. As shown, ESRP port count can be used to protect dual customer connections to the network. ESRP ping tracking is used to protect the core VLAN. In the exemplary embodiment shown, VRRP or HSRP protects the Carrier/ISP L3 switch.

### ESRP Enhancements

A preferred embodiment of the network includes network enhancements; including Extreme Network's ESRP, to support rapid failover of subscriber equipment when a network or core failure occurs. In the context of ESRP, this is referred to as "ESRP Failover Link Transition Enhancement." This enhancement refers to the ability of a ''Master'' ESRP switch, when transitioning to standby state to "bounce" or restart auto-negotiation on a set of physical ports. This enhancement will cause an end device to flush its Layer 2 forwarding database and cause it to re-broadcast immediately for a new path through the network. This provides the end station the ability to switch from the primary to the secondary path in a very short time.

This enhancement relates to the ability of a "Master" ESRP switch, when transitioning to a standby state to "bounce" or restart auto-negotiation on a set of physical ports. This is useful in this architecture to inform an end-user Layer 2 device of a failure farther within the network that does not directly impact the end-user Layer 2 device. As background: Typical Layer 3 switches use the Address Resolution Protocol (ARP) to populate their forwarding databases. This forwarding database determines which port packets are sent out on based on destination MAC address. Once this information is learned through the ARP process, typical Layer 2 devices will not modify this forwarding information unless one of two events occur. First, a Loss of Signal (LOS) occurs on the port or 2) the ARP max age timer expires. Typically, the ARP max age value is set to 5 minutes. When this timer expires, the Layer 2 device will re-ARP to update its forwarding database information. Therefore, if a failure occurs within the core of the network that does not cause a LOS on the end-user device, that device will continue to forward packets into the network even though they cannot reach their ultimate destination until the ARP max age timer expires. This is known as a black hole situation. The enhancement proposed here prevents a black hole situation, by notifying the end device of the core failure by "bouncing" the port to force the equipment to re-ARP to update its forwarding database information immediately.

Although certain preferred embodiments of the present invention have been described above by way of example, it will be understood that modifications may be made to the disclosed embodiments without departing from the scope of the invention, which is defined by the appended claims. The benefits, advantages, solutions to problems, and any element(s) that may cause any benefit, advantage, or solution to occur or become more pronounced are not to be construed as a critical, required, or essential feature or element of any or all the claims. As used herein, the terms "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus.

### Glossary of Terms

**ADM** - Add/Drop Multiplexer. A SONET component capable of inserting and removing traffic to/from the SONET line payload. ADMs also commonly perform other functions, such as generating/processing APS commands and synchronizing the transport optics to an external clock source.
**APS** - Automatic Protection Switching. A SONET fault recovery protocol standardized by Telcordia. APS will generally provide fault recovery in less than 50ms.
**BDN** - Built Distribution Network. A portion of a network dedicated to the aggregation of multiple subscribers. A BDN typically utilizes fiber to provide dedicated fiber links between individual subscribers and a Hub facility.
**BITS** - Building Integrated Timing Source. A highly accurate and precise clock source used to synchronize multiple nodes on a SONET transport system.
**Chromatic Dispersion** - A linear effect that causes pulse broadening or compression within an optical transmission system. Chromatic Dispersion is occurs because different wavelengths of light travel at different velocities through the transmission media.
**Collector Loop** - A fiber loop (typically 144ct) used to connect multiple subscribers to the larger Feeder loop on a BDN.
**Customer** - A business entity (such as an ISP or LEC) that provides telecommunications service within a Metropolitan area. The BDN operator typically will serve as an intermediate transport mechanism to connect subscribers to various customers.
**DMD** - Differential Mode Delay. A linear effect that degrades the quality of laser transmissions across MMF. A single laser transmission can inadvertently become subdivided upon ingress to MMF. These identical signals traverse unique transmission paths within the large core of MMF and leave the fiber offset in time.
**DS3** - Digital Signal 3. A digital signal rate of approximately 44.736 Mb/s corresponding to the North American T3 designator. A plesiochronous transport protocol equivalent to 672 voice lines at 64 kb/s each.
**DWDM** - Dense Wavelength Division Multiplexing. A method of allowing multiple transmission signals to be transmitted simultaneously over a single fiber by giving each a unique frequency range (or wavelength) within the transmission spectrum. DWDM wavelengths within the C-Band are standardized by the ITU-T.
**Ethernet** - A standardized (IEEE 802.3) packet-based data transport protocol developed by Xerox Corporation.
**Ethernet Switch** - A device used to route data packets to their proper destination in an Ethernet-based transport network.
**FDP** - Fiber Distribution Panel. An enclosure built to organize, manage, and protect physical cross-connections between multiple fiber-optic cables.
**Feeder Loop** - A fiber loop (typically 432ct) used to connect multiple collector loops to a Hub facility on a BDN.
**Fusion Splice** - The process of joining of two discrete fiber-optic cables via localized heating of the fiber ends. Fusion splices are typically characterized as permanent in nature and exhibit relatively minor loss (<.05dB) at the fusion point.
**Hub Facility -** A facility used to connect a distribution network (BDN or LDN) to a transport network (LTN) within a Metropolitan area.
**IR-1 -** A specification for transmission lasers and receiver photodiodes standardized by Telcordia. IR-1 optics typically provide a 13.0dB link budget and are optimized for NDSF.
**ITU-T** - International Telecommunications Union - Telecommunications Standardizations Sector.
**Lateral -** A fiber spur containing multiple fibers (e.g., 48ct) used to connect a Collector loop to a subscriber site.
**LDN** - Leased Distribution Network. A portion of a network dedicated to the aggregation of multiple subscribers. An LDN typically utilizes fiber to provide fiber links between individual subscribers and a Hub facility.
**LR-1** - A specification for transmission lasers and receiver photodiodes standardized by Telcordia. LR-1 optics typically provide a 25dB link budget and are optimized for NDSF.
**LTN** - Leased Transport Network. A portion of a network dedicated to connecting various Customer sites to Hub facilities. An LTN will typically utilize TDM and DWDM equipment over a small quantity of leased fiber.
**MCP -** Modal Conditioning Patch cord. A hybrid fiber-optic cable used to overcome DMD problems by allowing a laser to mimic the overfilled launch characteristics of an LED.
Mechanical Splice - The process of joining of two discrete fiber-optic cables by aligning them within a mechanical enclosure or adhesive media. Mechanical splices typically utilize an index-matching gel to reduce reflection at the splice point. Expect a moderate power loss (0.10 to 0.20dB) at the splice point
**Media Converter** - A generic classification of devices used to alter protocols and/or media of a transmitted signal.
**MMF** - Multi-Mode Fiber. A fiber-optic cable with a relatively large (50 to 62µm) transmission core that allows signals to traverse multiple, discrete transmission paths (modes) within the cable. MMF is typically utilized with LED-based optical transmission systems.
**Modal Distortion** - A linear effect that causes pulse broadening of transmission signals over MMF. Rays taking more direct paths (fewer reflections in the core) through the MMF core traverse the fiber more quickly than rays taking less direct paths. Modal distortion limits the bandwidth and distance of transmission links over MMF.
**MPOE** - Minimum Point of Entry. A common space within a multi-tenant building used to interconnect multiple tenants with common external telecommunications facilities.
**NDSF** - Non Dispersion-Shifted Fiber. Single-mode optical fiber with a nominal zero-dispersion wavelength within the conventional 1310nm transmission window.
**NZ-DSF -** Non-Zero Dispersion-Shifted Fiber. Single-mode optical fiber with a nominal zero-dispersion wavelength shifted to reduce chromatic dispersion within the 1530nm to 1560nm transmission window.
**OC-3** - Optical Carrier 3. The optical equivalent to an STS-3, with a digital signal rate of approximately 155.52 Mb/s. A synchronous transport protocol equivalent to 2016 voice lines at 64 kb/s each. Protocol is specified by Telcordia standards.
**OC-3c** - Optical Carrier 3, Concatenated. A non-channelized variant of the OC-3, primarily utilized for data transmissions over SONET. Protocol is specified by Telcordia standards.
**OC-12** - Optical Carrier 12. The optical equivalent to an STS-12, with a digital signal rate of approximately 622.08 Mb/s. A synchronous transport protocol equivalent to 8064 voice lines at 64 kb/s each. Protocol is specified by Telcordia standards.
**OC-12c** - Optical Carrier 12, Concatenated. A non-channelized variant of the OC-12, primarily utilized for data transmissions over SONET. Protocol is specified by Telcordia standards.
**OC-48** - Optical Carrier 48. The optical equivalent to an STS-48, with a digital signal rate of approximately 2.488 Gb/s. A synchronous transport protocol equivalent to 32256 voice lines at 64 kb/s each. Protocol is specified by Telcordia standards.
**OC-48c -** Optical Carrier 48, Concatenated. A non-channelized variant of the OC-48, primarily utilized for data transmissions over SONET. Protocol is specified by Telcordia standards.
**Plesiochronous** - The relationship between two transmission devices, where each is timed from similar, yet diverse clock sources. A slight difference in either frequency or phase must exist between the diverse clocks.
**POP** - Point of Presence. The physical facility in which interexchange carriers and local exchange carriers provide access services.
**SMF -** Single Mode Fiber. A type of optical fiber in which only a single transport path (mode) is available through the core at a given wavelength.
**SONET -** Synchronous Optical NETwork. A circuit-based transmission/restoration protocol defined by Telcordia standards. Use of the SONET TDM protocol is primarily limited to North America.
S**plice Box** - An enclosure built to organize, manage, and protect physical splices between multiple fiber-optic cables.
**SR** - A specification for transmission lasers and receiver photodiodes standardized by Telcordia. SR optics typically provide an 8dB link budget and are optimized for NDSF.
Subscriber - An end-user (or desired end-user) of a Customer's telecommunications service. A BDN operator typically will serve as an intermediate transport mechanism between subscribers and customers.
**Synchronous** - The relationship between two transmission devices, where both are timed from identical clock sources. The clocks must be identical in frequency and phase.
**TDM** - Time-Division Multiplexing. Combining multiple transmission signals into a common, higher-frequency bit-stream.
**WDM** - Wavelength-Division Multiplexing. A method of allowing multiple transmission signals to be transmitted simultaneously over a single fiber by giving each a unique frequency range (or wavelength) within the transmission spectrum.

## Claims

1. A network failover transition system for a plurality of ports communicating over a VLAN comprising:
a first switch (62) having a master mode and a standby mode, and configured to provide switching between said ports while in said master mode:
a second switch (63) having a master mode and a standby mode, and configured to provide switching between said ports while is said master mode, wherein said second switch (63) is in said standby mode when said first switch (62) is in said master mode, and said second switch (63) is in said master mode when said first switch (62) is in said standby mode;
wherein said second switch is configured, upon said detection of a network failure, to transition to said master mode,
**characterized in that**
said first switch is configured, upon a detection of a network failure, to restart auto-negotiation of said ports, and to transition to said standby mode.

2. The network failover transition system of claim 1, wherein said VLAN is part of an Ethernet Network.

3. The network failover transition system of claim 2, wherein said VLAN utilizes Extreme Networks Standby Router Protocol.

4. The network failover transition system of claim 3, wherein said first and second switches are Layer 2 switches.

5. The network failover transition system of claim 4, wherein said ports are Layer 3 devices.

6. The network failover transition system of claim 1, wherein at least one of said ports utilizes Address Resolution Protocol.

7. The network failover transition system of claim 1, wherein all of said ports utilize Address Resolution Protocol.

8. The network failover transition system of claim 1, wherein said network failure is detected using ping track.

9. The network failover transition system of claim 1, wherein said network failure is detected using port track.

10. A method of failover transitioning a VLAN with a plurality of ports comprising
establishing a first switch (62) having a master mode and a standby mode as a master switch;
configuring said master switch to provide switching between said ports;
establishing a second switch (63) having a master mode and a standby mode as a standby switch;
**characterized by** the steps of, upon detecting a communication failure on said VLAN, restarting auto-negotiation of said ports with said master switch, transitioning said first switch (62) to standby mode, whereby said first switch (62) becomes said standby switch, and transitioning said second switch (63) to master mode, whereby said second switch (63) becomes said master switch.

11. The method of failover transitioning a VLAN of claim 10, wherein said VLAN is part of an Ethernet Network.

12. The method of failover transitioning a VLAN of claim 11, wherein said VLAN utilizes Extreme Networks Standby Router Protocol.

13. The method of failover transitioning a VLAN of claim 12, wherein said first (62) and second (63) switches are Layer 2 switches.

14. The method of failover transitioning a VLAN of claim 13, wherein said ports are Layer 3 devices.

15. The method of failover transitioning a VLAN of claim 10, wherein at least one of said ports utilizes Address Resolution Protocol.

16. The method of failover transitioning a VLAN of claim 10, wherein all of said ports utilize Address Resolution Protocol.

17. The method of failover transitioning a VLAN of claim 10, wherein said detecting step comprises using ping track.

18. The method of failover transitioning a VLAN of claim 10, wherein said detecting step comprises using port track.

## Patentansprüche

1. Netzwerkausfallsicherung mit Umsprungssystem für eine Vielzahl von Anschlussstellen, die über ein VLAN kommunizieren, mit:
einem ersten Schalter (62), der einen Hauptmodus und einen Bereitschaftsmodus hat und so konfiguriert ist, dass er für das Schalten zwischen den Anschlussstellen sorgt, während er sich im Hauptmodus befindet:
einem zweiten Schalter (63), der einen Hauptmodus und einen Bereitschaftsmodus hat und so konfiguriert ist, dass er für das Schalten zwischen den Anschlussstellen sorgt, während er sich im Hauptmodus befindet, wobei der zweite Schalter (63) im Bereitschaftsmodus ist, wenn der erste Schalter (62) im Hauptmodus ist und der zweite Schalter (63) sich im Hauptmodus befindet, wenn der erste Schalter (62) im Bereitschaftsmodus ist;
wobei zweiter Schalter so konfiguriert ist, dass er bei der Detektion eines Netzwerkfehlers auf den Hauptmodus umspringt,
**dadurch gekennzeichnet, dass**
erster Schalter so konfiguriert ist, dass er bei der Detektion eines Netzwerkfehlers die automatische Verbindungsaushandlung (Autonegotiation) der Anschlussstellen wieder startet und in den Bereitschaftsmodus umspringt.

2. Netzwerkausfallsicherung mit Umsprungssystem gemäß Anspruch 1, wobei das VLAN Teil eines Ethernet Netzwerks ist.

3. Netzwerkausfallsicherung mit Umsprungssystem gemäß Anspruch 2, wobei das VLAN das Extreme Networks Notfall Router Protokoll benutzt.

4. Netzwerkausfallsicherung mit Umsprungssystem gemäß Anspruch 3, wobei der erste und zweite Schalter Schicht-2-Schalter sind.

5. Netzwerkausfallsicherung mit Umsprungssystem gemäß Anspruch 4, wobei die Anschlussstellen Schicht-3-Bauteile sind.

6. Netzwerkausfallsicherung mit Umsprungssystem gemäß Anspruch 1, wobei wenigstens eine der Anschlussstellen das Adressenzuordnungsprotokoll (Address Resolution Protocol ARP) benutzt.

7. Netzwerkausfallsicherung mit Umsprungssystem gemäß Anspruch 1, wobei alle Anschlussstellen das Adressenzuordnungsprotokoll verwenden.

8. Netzwerkausfallsicherung mit Umsprungssystem gemäß Anspruch 1, wobei das Netzwerkversagen detektiert wird, indem eine Ping-Spur verwendet wird.

9. Netzwerkausfallsicherung mit Umsprungssystem gemäß Anspruch 1, wobei das Netzwerkversagen detektiert wird, indem eine Anschlussstellen-Spur verwendet wird.

10. Verfahren zur Ausfallsicherungsumschaltung eines VLAN mit einer Vielzahl von Anschlussstellen, wobei ein erster Schalter (62) bereitgestellt wird, der als Hauptschalter einen Hauptmodus und einen Bereitschaftsmodus hat; der Hauptschalter konfiguriert wird, um zwischen den Anschlussstellen hin- und her zu schalten;
ein zweiter Schalter (63) als Bereitschaftsschalter bereitgestellt wird, der einen Hauptmodus und einen Bereitschaftsmodus hat;
**dadurch gekennzeichnet, dass** bei Detektion eines Kommunkationsversagens bei dem VLAN die folgende Schritte ausgeführt werden: Wiederaufnahme der automatischen Verbindungsaushandlung der Anschlussstellen mit dem Hauptschalter, Umschalten des ersten Schalters (62) in den Bereitschaftsmodus, wodurch der erste Schalter (62) Bereitschaftsschalter wird, und Umschalten des zweiten Schalters (63) in den Hauptmodus, wodurch der zweite Schalter (63) Hauptschalter wird.

11. Verfahren zur Ausfallsicherungsumschaltung eines VLAN gemäß Anspruch 10, wobei das VLAN Teil eines Ethernet Netzwerks ist.

12. Verfahren zur Ausfallsicherungsumschaltung eines VLAN gemäß Anspruch 11, wobei das VLAN das Extreme Networks Notfall Router Protokoll benutzt.

13. Verfahren zur Ausfallsicherungsumschaltung eines VLAN gemäß Anspruch 12, wobei der erste (62) und zweite (63) Schalter Schicht-2-Schalter sind.

14. Verfahren zur Ausfallsicherungsumschaltung eines VLAN gemäß Anspruch 13, wobei die Anschlussstellen Schicht-3-Bauteile sind.

15. Verfahren zur Ausfallsicherungsumschaltung eines VLAN gemäß Anspruch 10, wobei wenigstens eine der Anschlussstellen das Adressenzuordnungsprotokoll (ARP) benutzt.

16. Verfahren zur Ausfallsicherungsumschaltung eines VLAN gemäß Anspruch 10, wobei alle Anschlussstellen das Adressenzuordnungsprotokoll (ARP) benutzen.

17. Verfahren zur Ausfallsicherungsumschaltung eines VLAN gemäß Anspruch 10, wobei der Detektionsschritt das Benutzen der Ping-Spur einschließt.

18. Verfahren zur Ausfallsicherungsumschaltung eines VLAN gemäß Anspruch 10, wobei der Detektionsschritt das Benutzen der Anschlussstellen-Spur einschließt.

## Revendications

1. Système de transition de basculement réseau pour une pluralité de ports communiquant sur un réseau VLAN (réseau local virtuel) comprenant :
un premier commutateur (62) ayant un mode maître et un mode veille, et configuré pour permettre une commutation entre lesdits ports lorsqu'il est en mode maître ;
un second commutateur (63) ayant un mode maître et un mode veille, et configuré pour permettre une commutation entre lesdits ports lorsqu'il est dans ledit mode maître, dans lequel ledit second commutateur (63) se trouve dans ledit mode veille lorsque ledit premier commutateur (62) se trouve dans ledit mode maître, et ledit second commutateur (63) se trouve dans ledit mode maître lorsque ledit premier commutateur (62) se trouve dans ledit mode veille ;
ledit second commutateur étant configuré, lors de ladite détection d'un basculement réseau, pour une transition vers ledit mode maître,
**caractérisé en ce que**
ledit premier commutateur est configuré, lors d'une détection d'un basculement réseau, pour redémarrer l'auto-négociation desdits ports, et pour une transition vers ledit mode veille.

2. Système de transition de basculement réseau selon la revendication 1, dans lequel ledit réseau VLAN fait partie d'un réseau Ethernet.

3. Système de transition de basculement réseau selon la revendication 2, dans lequel ledit réseau VLAN utilise le protocole de routage en veille d'Extreme Networks (Extreme Networks Standby Router Protocol).

4. Système de transition de basculement réseau selon la revendication 3, dans lequel lesdits premier et second commutateurs sont des commutateurs de couche 2.

5. Système de transition de basculement réseau selon la revendication 4, dans lequel lesdits ports sont des dispositifs de couche 3.

6. Système de transition de basculement réseau selon la revendication 1, dans lequel au moins l'un desdits ports utilise le protocole de résolution d'adresse.

7. Système de transition de basculement réseau selon la revendication 1, dans lequel tous les ports utilisent le protocole de résolution d'adresse.

8. Système de transition de basculement réseau selon la revendication 1, dans lequel ledit échec réseau est détecté en utilisant le suivi de ping.

9. Système de transition de basculement réseau selon la revendication 1, dans lequel ledit échec réseau est détecté en utilisant le suivi de port.

10. Procédé de transition de basculement d'un réseau VLAN avec une pluralité de ports comprenant
l'établissement d'un premier commutateur (62) ayant un mode maître et un mode veille en tant que commutateur maître ;
la configuration dudit commutateur maître pour permettre une commutation entre lesdits ports ;
l'établissement d'un second commutateur (63) ayant un mode maître et un mode veille en tant que commutateur veille ;
**caractérisé par** les étapes de , lors de la détection d'un échec de communication sur ledit réseau VLAN, redémarrage de l'auto-négociation desdits ports avec ledit commutateur maître, transition dudit premier commutateur (62) au mode veille, moyennant quoi ledit premier commutateur (62) devient ledit commutateur veille, et de transition dudit second commutateur (63) au mode maître, moyennant quoi ledit second commutateur (63) devient ledit commutateur maître.

11. Procédé de transition de basculement d'un réseau VLAN selon la revendication 10, dans lequel le réseau VLAN fait partie d'un réseau Ethernet.

12. Procédé de transition de basculement d'un réseau VLAN selon la revendication 11, dans lequel ledit réseau VLAN utilise le protocole de routage en veille d' Extreme Networks (Extreme Networks Standby Router Protocol).

13. Procédé de transition de basculement d'un réseau VLAN selon la revendication 12, dans lequel lesdits premier (62) et second (63) commutateurs sont des commutateurs de couche 2.

14. Procédé de transition de basculement d'un réseau VLAN selon la revendication 13, dans lequel lesdits ports sont des dispositifs de couche 3.

15. Procédé de transition de basculement d'un réseau VLAN selon la revendication 10, dans lequel au moins l'un desdits ports utilise le protocole de résolution d'adresse.

16. Procédé de transition de basculement d'un réseau VLAN selon la revendication 10, dans lequel tous les ports utilisent le protocole de résolution d'adresse.

17. Procédé de transition de basculement d'un réseau VLAN selon la revendication 10, dans lequel ladite étape de détection comprend l'utilisation du suivi de ping.

18. Procédé de transition de basculement d'un réseau VLAN selon la revendication 10, dans lequel ladite étape de détection comprend l'utilisation du suivi de port.
